# FASCICULE DE BREVET EUROPEEN

(11) **EP 3 797 169 B1**
(45) Date de publication et mention de la délivrance du brevet: **20.07.2022**
(21) Numéro de dépôt: 19752728.6
(22) Date de dépôt: 14.03.2019
(51) Int. Cl.: C12Q 1/04, C12M 1/34, C12Q 1/18

(54) **PROCÉDÉ DE CONTRÔLE DE SENSIBILITÉ À DES AGENTS CHIMIQUES**
VERFAHREN ZUR PRÜFUNG DER EMPFINDLICHKEIT GEGENÜBER CHEMIKALIEN
METHOD FOR TESTING SENSITIVITY TO CHEMICAL AGENTS

(43) Date de publication de la demande: 31.03.2021
(73) Titulaire: Hitachi High-Tech Corporation, Minato-ku Tokyo 105-6409 (JP); IHU Mediterranee Infection, 13385 Marseille Cédex 05 (FR)
(72) Inventeur: MATSUBARA, Shigeki, TOKYO, Tokyo 105--8717 (JP); OOMINAMI, Yuusuke, TOKYO, Tokyo 105--8717 (JP); IMAI, Kyoko, TOKYO, Tokyo 105--8717 (JP); IRIE, Takashi, TOKYO, Tokyo 105--8717 (JP); RAOULT, Didier, 13385 MARSEILLE Cédex 05 (FR); BOU KHALIL, Jacques, 13385 MARSEILLE Cédex 05 (FR)
(74) Mandataire: Nony
(86) Numéro de dépôt international: PCT/FR2019/050576
(87) Numéro de publication internationale: WO 2020/183072

(56) Documents cités:
- EP-A1- 2 757 371
- EP-A1- 3 121 262
- EP-A1- 3 252 138
- EP-A1- 3 287 515
- WO-A1-01/09371
- WO-A1-2014/145899
- WO-A1-2015/189390
- WO-A1-2018/136864
- WO-A2-2012/151563
- JP-A- 2005 261 260
- US-A1- 2006 134 728

## Description

### DOMAINE TECHNIQUE

La présente invention se rapporte à un procédé de contrôle de sensibilité à des agents chimiques.

### ART ANTERIEUR

Un appareil de contrôle permettant l'acquisition d'images de colonies de microbes dans une boîte de culture et la mesure de microbes et de cellules, et similaires, a été développé dans le but de faciliter l'automatisation et l'allégement de travail de la séparation de culture destinée à être utilisée dans des laboratoires d'essais (PTL 1). De plus, afin de raccourcir le temps requis pour le contrôle, il a été décrit un appareil de contrôle, lequel comprend une pluralité de puits dans une plaque de culture qui contient un liquide de culture contenant un microbe ou un champignon dans chaque puits et observe microscopiquement le microbe ou le champignon dans le liquide de culture contenu dans chaque puits (PTL 2). PTL 3 décrit un instrument permettant d'effectuer un test de sensibilité de bactéries à des agents antimicrobiens. L'instrument comprend un système optique d'observation microscopique avec une pluralité de puits, qui effectue une observation des bactéries à une pluralité de moments, et un processeur configuré pour afficher des images obtenues par observation microscopique

### Liste de citations

### Documents de brevet

PTL 1 : JP-A-2005-261260.3
PTL 2 : JP-A-2015-177768
PTL 3 : EP 3252138 A1

### RESUME DE L'INVENTION

### Problème technique

Cependant, dans l'appareil selon le document de brevet 1, étant donné qu'il est nécessaire que le microbe croisse suffisamment pour être évalué, dans le cas d'un microbe à croissance lente tel que *Pseudomonas aeruginosa,* il faut huit heures ou plus pour la culture jusqu'à ce qu'une seule colonie soit obtenue.

De plus, dans l'appareil selon le document de brevet 2, bien que la durée du contrôle soit raccourcie par l'utilisation de l'observation microscopique d'un microbe qui évalue la forme de chaque microbe, étant donné qu'un dispositif dédié ayant un mécanisme compliqué et une plaque de culture dédiée sont requis, il est inévitable que le coût du contrôle soit accru.

Par conséquent, un objet de la présente invention consiste à fournir un procédé de contrôle qui nous permet d'évaluer une sensibilité d'un microbe à des agents chimiques rapidement, de manière polyvalente et à faible coût.

### Solution au problème

Le procédé de contrôle permettant le contrôle de la sensibilité d'un microbe à un agent anti-microbien, comprenant : une première étape consistant à préparer un premier échantillon mélangé d'un liquide contenant un microbe avec l'agent anti-microbien ; une deuxième étape consistant à maintenir le premier échantillon chaud à une température prédéfini et à maintenir chaud à une température prédéfinie un second échantillon qui est un liquide qui contient un second microbe qui est la même souche que le premier microbe sans l'agent anti-microbien ; une troisième étape consistant à observer un premier microbe dans le premier échantillon par un microscope à une pluralité de moments prédéfinis qui comprennent un état initial dans lequel le premier microbe n'est pas influencé par l'agent anti-microbien et a observer un second microbe par le microscope à au moins un de la pluralité de moments prédéfinis , une quatrième étape consistant à analyser l'aspect du premier microbe observé et à comparer une première image concernant un aspect du premier microbe avec une seconde image concernant un aspect du second microbe à au moins un de la pluralité de moments prédéfinis ; et une cinquième étape consistant à évaluer la sensibilité à des agents chimiques du premier microbe vis-à-vis de l'agent anti-microbien sur la base d'un changement d'aspect du premier microbe observé dans lequel le microscope est un microscope électronique à balayage ou un microscope sonde à balayage.

### Effets avantageux de l'invention

Selon la présente invention, il est possible de fournir un procédé de contrôle qui accélère une évaluation d'une sensibilité d'un microbe à des agents chimiques.

### BREVE DESCRIPTION DES DESSINS

[Fig. 1] La fig. 1 est un schéma illustrant une suite d'opérations d'évaluation d'un procédé de contrôle d'une sensibilité d'un microbe à des agents chimiques.
[Fig. 2] La fig. 2 est un schéma illustrant une suite détaillée d'opérations d'évaluation d'un procédé de contrôle selon un premier mode de réalisation.
[Fig. 3] La fig. 3 est un schéma illustrant une suite détaillée d'opérations d'évaluation d'un procédé de contrôle selon un deuxième mode de réalisation.
[Fig. 4] La fig. 4 est un schéma illustrant des microbes et des antibiotiques à titre de sujets principaux.
[Fig. 5] La fig. 5 est une vue illustrant l'exemple 1 d'images au microscope électronique.
[Fig. 6] La fig. 6 est une vue illustrant l'exemple 2 d'images au microscope électronique.

### DESCRIPTION DE MODES DE REALISATION

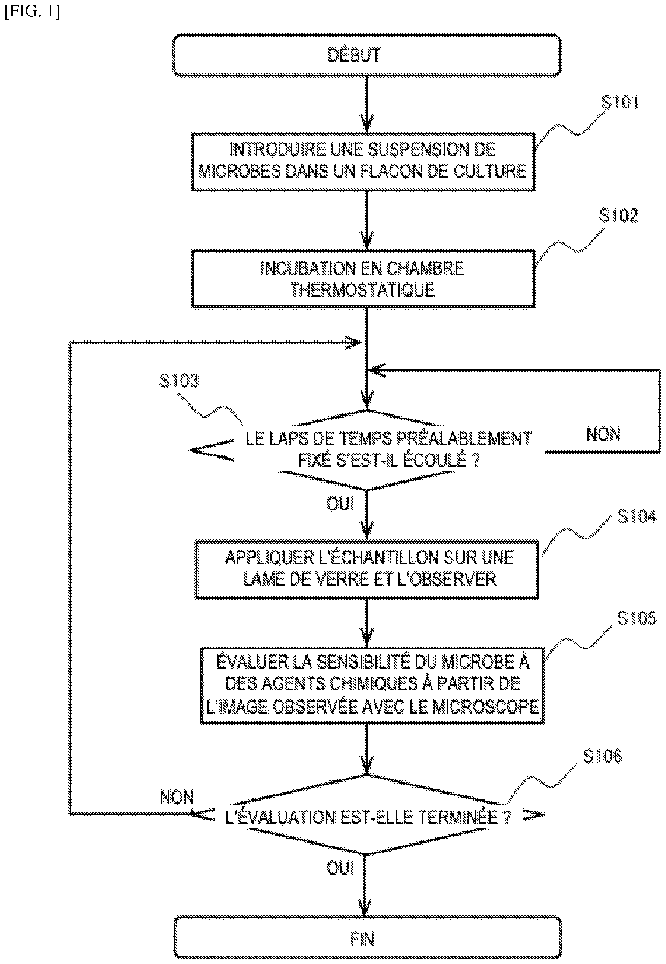

Ci-après, des exemples vont être décrits en référence aux dessins ci-joints. La fig. 1 est un schéma illustrant une suite d'opérations d'évaluation d'un procédé de contrôle d'une sensibilité d'un microbe à des agents chimiques.

Tel qu'utilisé ici, un test de sensibilité microbienne à des agents chimiques fait référence à un contrôle pour savoir si les mêmes souches sont pharmacorésistantes ou non sur la base des conditions dans lesquelles les microbes sont amenés à proliférer dans une culture de microbes cultivés dans un milieu de culture contenant divers agents antimicrobiens à une concentration prédéfinie ou à l'évaluation de la concentration minimale inhibitrice (CMI) du microbe. Ici, le terme « microbe » inclut les termes « bactéries » et « champignons ». En outre, les bactéries faisant l'objet d'un contrôle par ce procédé ne sont pas particulièrement limitées. Par exemple, les bactéries comprennent *Staphylococcus aureus, Enterococci, Pneumococcus, Escherichia coli, Pseudomonas aeruginosa, Klebsiella pneumoniae* et similaires.

Lors de la réalisation d'un test selon ce procédé, une suspension de microbes est souvent préparée à l'aide d'une seule colonie obtenue à partir d'un échantillon clinique par culture séparée. D'un autre côté, lorsque l'échantillon clinique présente un faible risque de contamination et ne contient qu'un seul microbe, il peut être utilisé tel quel ou dilué de manière appropriée sans préparation d'une suspension de microbes. De plus, il est souhaitable de suivre la méthode recommandée par le *Clinical and Laboratory Standards Institute* (CLSI) pour le prélèvement et le transport d'échantillons, la culture de séparation, la préparation d'agent antimicrobien, la préparation de milieu, la température de culture, le liquide de culture et similaires, mais sans y être limité.

Ensuite, la procédure de culture va être décrite. D'abord, une suspension de microbes préparée à partir d'un échantillon est introduite dans un flacon de culture (ci-dessous appelé « flacon ») (S101). Ensuite, la suspension de microbes mélangée avec le liquide de culture contenant à chaque fois différents agents antimicrobiens à une concentration particulière dans chaque flacon est incubée et cultivée dans une chambre thermostatique réglée à environ 35 °C (S102). À ce moment-là, un échantillon mélangé avec le liquide de culture sans agent antimicrobien est cultivé en même temps en tant qu'échantillon témoin. Ensuite on détermine si un laps de temps préalablement fixé prédéfini s'est écoulé (S103). Lorsque le laps de temps prédéfini s'est écoulé, le flacon est sorti de la chambre thermostatique et un échantillon du contenu du flacon contenant le liquide de culture mélangé avec la suspension de microbes est appliqué sur une lame de verre et observé avec un microscope. À ce moment-là, un prétraitement tel qu'une coloration ou similaire n'est pas nécessairement requis. Ensuite, la sensibilité à des agents chimiques (ci-après appelée « sensibilité ») du microbe est évaluée à partir de l'image microscopique (S104). Par la suite, on détermine si l'évaluation doit être terminée ou non (S106) et si on détermine que la détermination doit être terminée, le processus est terminé.

L'observation peut être effectuée à des intervalles de temps préalablement fixés du début à la fin de l'incubation et l'aspect de la prolifération de microbes peut être suivi en continu. De plus, une durée appropriée peut être fixée, auquel cas un suivi peut être effectué au bout de la durée fixée et comparé avec un résultat de suivi obtenu au début de l'incubation. De plus, une pluralité de flacons de culture contenant une suspension de microbes et une concentration particulière d'agent antimicrobien peuvent être préparés à l'avance et la suspension de microbes contenue dans différents flacons peut être observée à chaque fois qu'un laps de temps prédéfini s'est écoulé. De plus, la concentration de l'agent antimicrobien peut ne pas être spécifiée et un rapport de la suspension de microbes et d'un agent antimicrobien peut être spécifié.

### EXEMPLES

### Exemple 1

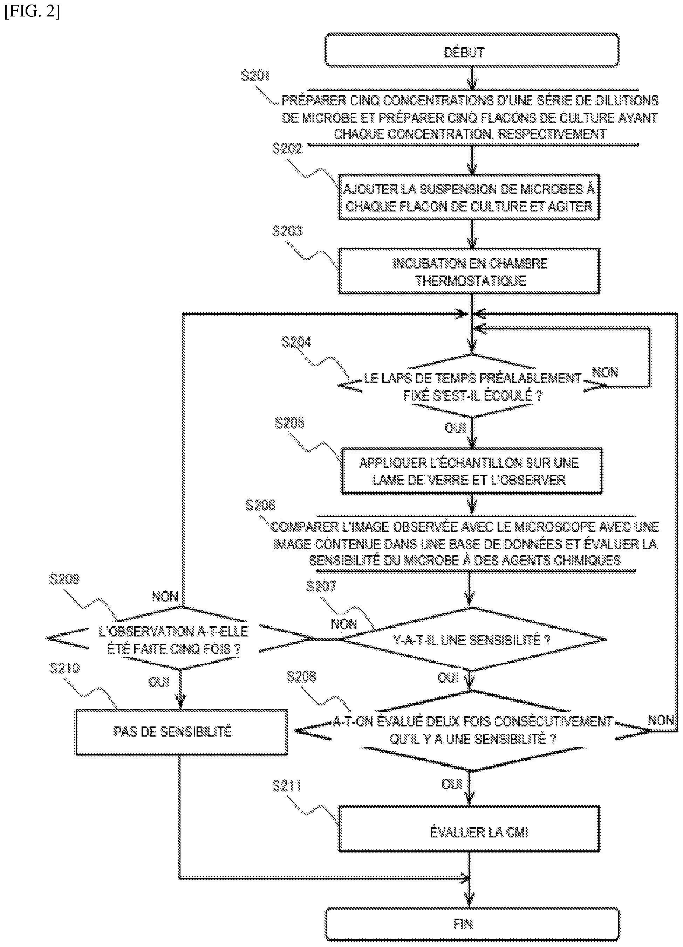

La fig. 2 est un schéma montrant une suite détaillée d'opérations d'évaluation d'un procédé de contrôle selon le premier mode de réalisation. Dans l'exemple 1, une pluralité d'images de microbes qui ont été confirmés être des microbes résistants et une pluralité d'images de microbes qui ont été confirmés avoir une sensibilité à un agent antimicrobien sont introduites dans une base de données.

D'abord, cinq concentrations d'une série de dilutions d'agent antimicrobien sont préparées et cinq flacons de chaque concentration sont préparés (S201). Cela consiste à permettre d'observer chaque concentration de la série cinq fois à des intervalles de temps prédéfinis (par exemple toutes les 30 minutes). Cependant, les intervalles de temps d'observation et le nombre d'observations ne sont pas limités à ceux ci-dessus. Ensuite, la suspension de microbes est introduite dans chaque flacon de culture et agitée (S202) et les 25 flacons sont placés dans une chambre thermostatique réglée à 35 °C et incubés dans la chambre thermostatique (S203). Ensuite, on détermine si un laps de temps préalablement fixé prédéfini s'est écoulé (S204). Lorsque le laps de temps prédéfini s'est écoulé, chacun des flacons aux 5 concentrations est sorti individuellement et l'échantillon du contenu du flacon sorti est appliqué sur une lame de verre et observé (S205). On évalue alors si le microbe est un microbe résistant ou a une sensibilité par comparaison des images microscopiques avec les images contenues dans la base de données (S206).

Ici, on évalue si le microbe a une sensibilité (S207) et si on évalue que le microbe a une sensibilité, on évalue à nouveau (deux fois consécutivement) si le microbe a une sensibilité (S208). Lorsqu'on évalue les deux fois que l'échantillon contenant la concentration d'agent antimicrobien particulier a une sensibilité, la plus faible concentration d'agent antimicrobien parmi les échantillons qui sont évalués comme ayant une sensibilité est évaluée être la CMI (S211) et le processus est terminé.

En S207, lorsqu'on évalue que le microbe n'a pas de sensibilité, on détermine si l'observation a été faite cinq fois (S209). Lorsqu'on détermine que l'observation a été faite cinq fois, on évalue qu'il n'y a pas de sensibilité (S210) et le processus est terminé et si on détermine que l'observation n'a pas été faite cinq fois, le processus retourne à S204.

En outre, lorsqu'on détermine qu'il n'y a pas de sensibilité en S207 ou S208, ou après que le laps de temps prédéfini s'est écoulé, de manière similaire, une image observée avec un microscope électronique est acquise et comparée avec l'image de la base de données pour évaluer si le microbe est le microbe résistant ou a la sensibilité. Par la suite, les images observées sont acquises à chaque fois que le laps de temps prédéfini s'est écoulé. Lorsqu'on détermine que l'échantillon a une sensibilité à toutes les concentrations d'agent antimicrobien, ou lorsque l'observation des échantillons du contenu de tous les flacons est terminée, la plus faible concentration d'agent antimicrobien parmi les échantillons évalués comme ayant une sensibilité est évaluée être la CMI.

Un logiciel capable de discrimination automatique peut être utilisé pour comparer les images contenues dans la base de données avec les images acquises avec le microscope électronique. Le logiciel permet de générer automatiquement des algorithmes de discrimination par apprentissage mécanique des caractéristiques d'images contenues dans la base de données. De plus, à un moment approprié, les images du microbe qui a été confirmé être un microbe résistant et/ou les images de microbe confirmé avoir une sensibilité à un agent antimicrobien peuvent de plus être enregistrées dans la base de données. En outre, le logiciel permet de mettre à jour des algorithmes de discrimination par apprentissage mécanique des caractéristiques d'images concluantes enregistrées de plus dans la base de données, en continu.

### Exemple 2

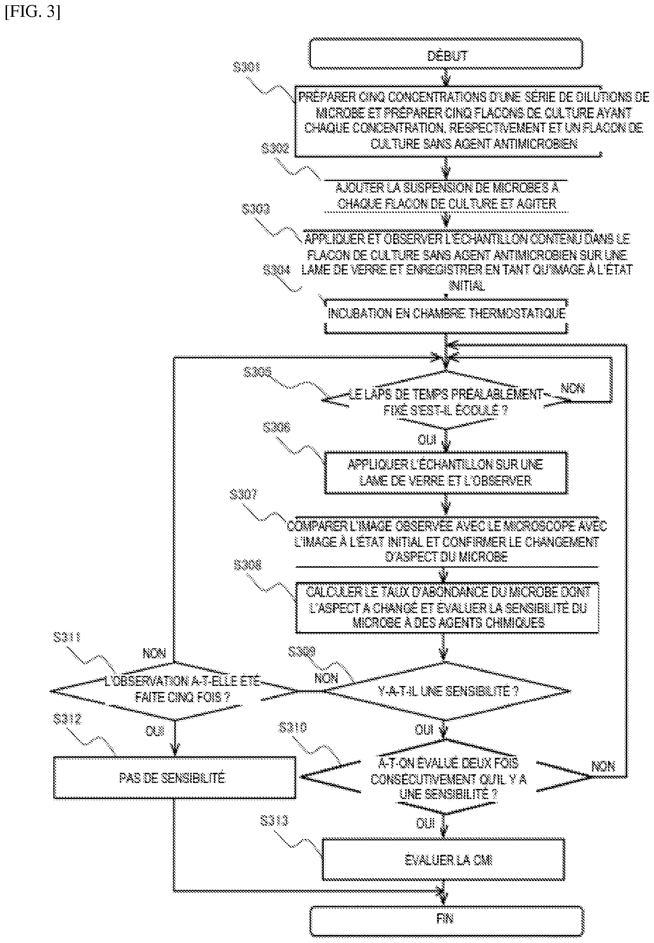

La fig. 3 est un schéma montrant une suite détaillée d'opérations d'évaluation d'un procédé de contrôle selon un deuxième mode de réalisation.

D'abord, cinq concentrations d'une série de dilutions d'agent antimicrobien sont préparées et cinq flacons de chaque concentration sont préparés (S301). L'intervalle de temps entre les observations et le nombre d'observations sont les mêmes que dans l'exemple 1. Ensuite, la suspension de microbes est introduite dans chaque flacon et agitée (S302) et un échantillon du contenu du flacon ne contenant pas d'agent antimicrobien est appliqué sur une lame de verre et observé avec le microscope électronique pour obtenir une image dans un état initial (S303).

Ensuite, les 25 flacons sont placés dans une chambre thermostatique réglée à 35 °C et incubés dans la chambre thermostatique (S304). Au bout d'une durée prédéfinie (S305), chacun des flacons aux cinq concentrations est sorti individuellement et l'échantillon du contenu du flacon est appliqué sur une lame de verre et observé avec le microscope électronique (S306). En outre, des informations d'aspect du microbe entier, qui sont obtenues par compilation de données d'aspect du microbe observé, et un nombre total des microbes dans la pluralité de champs sont enregistrés et une image acquise par l'observation au microscope électronique est comparée avec l'image à l'état initial pour confirmer s'il y a un microbe dont l'aspect a changé (S307).

Ici, l'aspect du microbe désigne une forme du microbe et/ou une luminosité de la zone où le microbe est présent. Par exemple, la forme peut inclure la rondeur, le rapport grand axe/petit axe, la surface d'occupation, la surface d'agrégats formés par une pluralité de microbes, le nombre de microbes à l'intérieur de l'agrégat, la densité microbienne à l'intérieur de l'agrégat et similaires et la luminosité peut inclure, par exemple, un rapport de contraste et similaire. De plus, la forme peut être une forme d'un agrégat lorsqu'une pluralité des microbes forment l'agrégat et/ou une forme de chaque microbe qui est indépendant de l'agrégat.

La situation où les zones dont la luminosité est élevée (taches hyper-denses) apparaissent pendant la division cellulaire de microbes est confirmée. Par exemple, lorsque la division cellulaire d'un bacille commence, la luminosité de la périphérie du bacille, en particulier des deux extrémités du grand axe, devient plus élevée. Si la division se poursuit encore plus, la luminosité de parties de division générées au voisinage du centre de la cellule d'origine devient également plus élevée. De cette manière, si nous observons le changement de luminosité, alors nous pouvons confirmer que l'aspect de microbes a changé.

Lorsqu'il y a des microbes dont l'aspect a changé, le nombre des microbes dont l'aspect a changé présents dans le champ est enregistré et un taux d'abondance des microbes dont l'aspect a changé est calculé à partir du nombre total de microbes et du nombre de microbes dont l'aspect a changé.

Lorsque le taux d'abondance calculé dépasse une valeur prédéfinie fixée à l'avance, on évalue que le microbe a une sensibilité à un agent antimicrobien. De plus, lorsque 60 minutes se sont écoulées, une image observée avec le microscope électronique est acquise et le taux d'abondance du nombre de microbes dont l'aspect a changé est calculé pour évaluer si le microbe est le microbe résistant ou a la sensibilité (S308). Les processus suivants sont les mêmes que dans l'exemple 1.

Un seul échantillon peut être observé, auquel cas des images dans des champs respectifs sont acquises, le nombre total de microbes et le nombre de microbes dont l'aspect a changé dans chaque champ sont enregistrés, le nombre de microbes dans tous les champs est totalisé et le taux d'abondance de microbes dont l'aspect a changé peut être calculé. Il est souhaitable que le nombre total de tous les microbes soit supérieur ou égal à 1 000, bien qu'il ne soit pas limité à ce chiffre.

Quand on évalue si un microbe est un microbe résistant ou non, lorsque la variation du taux d'abondance calculé au cours du temps (par exemple la variation au cours du temps par rapport à l'état initial du taux d'abondance) dépasse une certaine valeur fixée à l'avance, on peut déterminer que le microbe a une sensibilité à un agent antimicrobien. De plus, une combinaison de méthodes d'évaluation peut être utilisée. Par exemple, une évaluation peut être faite sur la base du taux d'abondance après que 30 minutes se sont écoulées et ensuite faite sur la base du taux de variation du taux d'abondance après que 60 minutes se sont écoulées.

De plus, des informations d'aspect dans le cas où l'agent antimicrobien est donné à un microbe qu'une personne prédéfinie a, peuvent être comparées avec des informations d'aspect dans le cas où l'agent antimicrobien n'est pas donné à un microbe que la personne prédéfinie a et la sensibilité à l'agent antimicrobien du microbe que la personne prédéfinie a peut être évaluée sur la base du résultat de la comparaison.

De plus, on peut obtenir un critère concernant le taux d'abondance de changement d'aspect, établi sur la base d'un agent antimicrobien d'un microbe standard prédéfini et d'une concentration prédéfinie et la sensibilité d'un microbe qu'une personne prédéfinie a peut être évaluée sur la base du critère.

Pour la discrimination de microbe dont l'aspect a changé dans l'image, un logiciel capable de discrimination automatique peut être utilisé. Le logiciel permet de générer automatiquement des algorithmes de discrimination par apprentissage mécanique des caractéristiques de changements d'aspect à partir d'une base de données d'images de microbe dans un état initial préparée à l'avance et à partir d'une base de données d'images de microbe dont l'aspect a changé en raison de l'influence d'un agent antimicrobien. De plus, un logiciel permettant l'évaluation et l'enregistrement automatiques du nombre de microbes peut être utilisé.

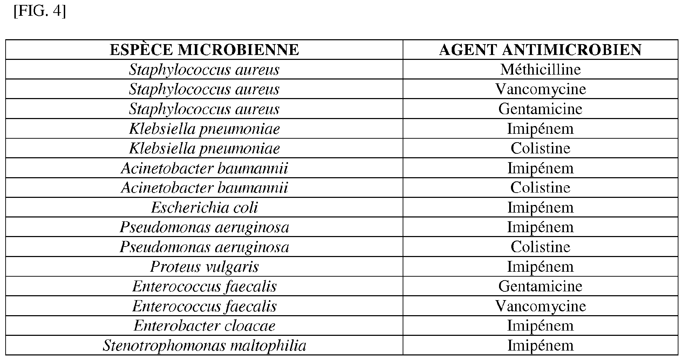

La fig. 4 est un schéma illustrant des microbes et des antibiotiques à titre de sujets principaux. Les caractéristiques d'aspect qui sont indiquées plus loin donnent des indications qui permettent de détecter la sensibilité ou la résistance à un agent antimicrobien. Ci-dessous se trouvent quelques exemples tels que confirmés jusqu'ici.

Chez un microbe à gram négatif, une forte réfraction ou un grand angle de réfraction d'un faisceau d'électrons sont observés aux deux extrémités de la division cellulaire pendant la phase de croissance. Lorsque des microbes ont une sensibilité à un agent antimicrobien destiné à être contrôlé, il y a peu de microbes présentant un aspect réfractif du faisceau d'électrons et le rapport entre les microbes ne présentant pas de réfraction et les microbes présentant une réfraction est perceptible à l'état initial. À un stade ultérieur, les microbes sensibles présentent un allongement des cellules, trois branches, deux branches, une micronisation et une sphéronisation.

Chez les coques à gram positif tels que les staphylocoques, il a été observé que le microbe sensible a un corps agrandi.

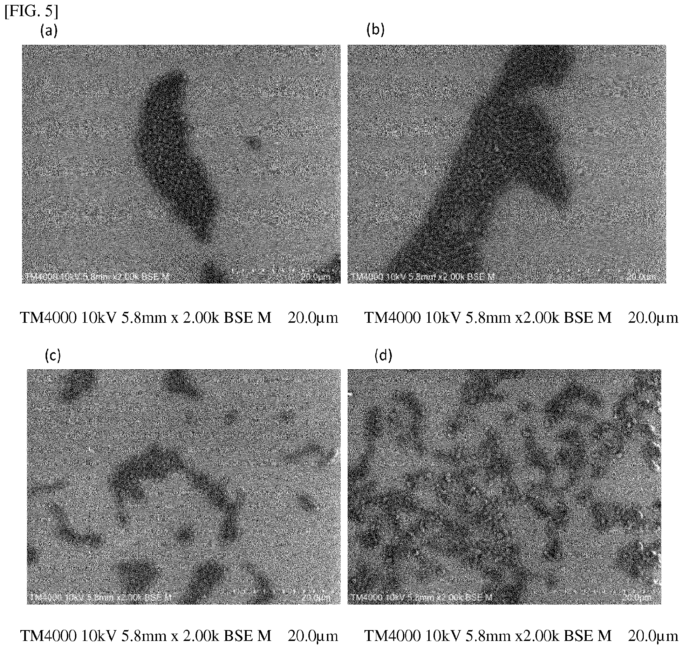

La fig. 5 est une vue montrant l'exemple 1 d'une image au microscope électronique montrant un résultat de culture *d'Escherichia coli* dans un liquide de culture contenant 1 mg/l d'imipénem. Dans la fig. 5, (a) et (b) montrent des images de microbes confirmés être résistants à l'imipénem, (c) et (d) montrent des images de microbes confirmés avoir une sensibilité à l'imipénem et (a) à (d) montrent les états 30 minutes, 120 minutes, 30 minutes et 120 minutes après le début de la culture, respectivement. Les microbes résistants aussi bien dans (a) que dans (b) forment des agrégats, mais l'agrégat dans (c) est très petit et dans (d), aucun agrégat n'est formé. De plus, la luminosité des microbes dans (c) et (d) est plus élevée que celle dans (a) et (b). Bien qu'il soit possible de confirmer le contour du microbe sans coloration à l'aide du microscope optique, il est difficile d'obtenir des informations d'aspect plus détaillées pour un microbe individuel.

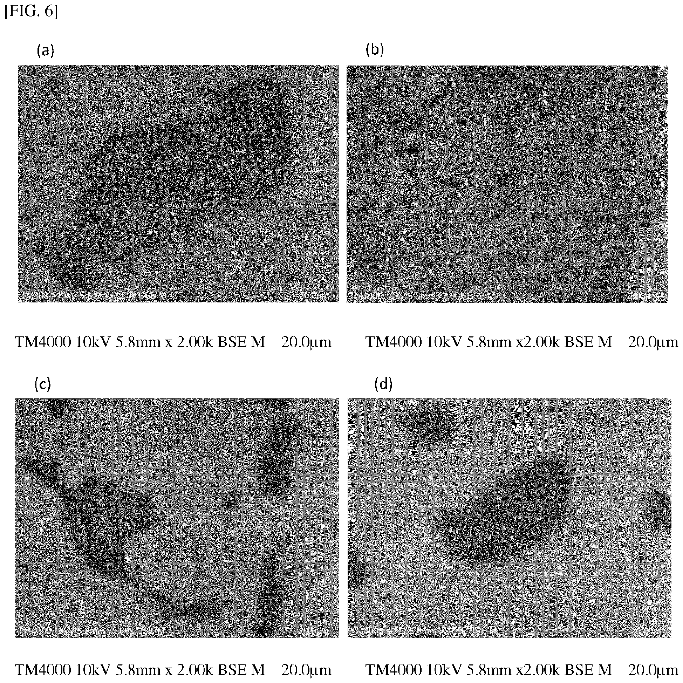

La fig. 6 est une vue montrant l'exemple 2 d'une image au microscope électronique montrant un résultat de culture de *Klebsiella pneumoniae* dans un liquide de culture contenant 1 mg/l d'imipénem. Dans la fig. 6, (a) et (b) montrent des images de microbes confirmés être résistants à l'imipénem, (c) et (d) montrent des images de microbes confirmés avoir une sensibilité à l'imipénem et (a) à (d) montrent les états 30 minutes, 120 minutes, 30 minutes et 120 minutes après le début de la culture, respectivement. On peut confirmer que dans (a) et (b), la densité de l'agrégat formé par les microbes est faible, tandis que dans (c) et (d), l'agrégat est formé dans un état de densité élevée. De plus, on peut confirmer que, alors que (a) et (b) montrent une croissance par division chez de nombreux microbes, dans (c) et (d), il n'y a pas de division et chaque microbe a une forme d'aspect présentant un grand axe raccourci en une forme proche d'un cercle. On peut également confirmer que la luminosité des microbes dans (c) et (d) est plus faible que celle dans (a) et (b).

Dans le présent mode de réalisation, il est décrit que l'observation est effectuée à l'aide d'un microscope électronique, mais, par exemple, le microscope électronique peut faire référence à un microscope électronique à balayage (MEB), un microscope électronique à balayage de table ou un microscope électronique à balayage qui permet une observation à pression atmosphérique ou un microscope-sonde à balayage.

## Revendications

1. Procédé de contrôle permettant le contrôle de la sensibilité d'un microbe à un agent antimicrobien, comprenant :
une première étape consistant à préparer un premier échantillon mélangé d'un liquide contenant un microbe avec l'agent antimicrobien ;
une deuxième étape consistant à maintenir le premier échantillon chaud à une température prédéfinie et à maintenir chaud à une température prédéfinie un second échantillon qui est un liquide qui contient un second microbe qui est la même souche que le premier microbe sans l'agent antimicrobien ;
une troisième étape consistant à observer un premier microbe dans le premier échantillon par un microscope à une pluralité de moments prédéfinis qui comprennent un état initial dans lequel le premier microbe n'est pas influencé par l'agent antimicrobien et à observer un second microbe par le microscope à au moins un de la pluralité de moments prédéfinis ;
une quatrième étape consistant à analyser l'aspect du premier microbe observé et à comparer une première image concernant un aspect du premier microbe avec une seconde image concernant un aspect du second microbe à au moins un de la pluralité de moments prédéfinis ; et
une cinquième étape consistant à évaluer la sensibilité à des agents chimiques du premier microbe vis-à-vis de l'agent antimicrobien sur la base d'un changement d'aspect du premier microbe observé,
dans lequel le microscope est un microscope électronique à balayage ou un microscope sonde à balayage.

2. Procédé de contrôle selon la revendication 1, dans lequel
la première étape consiste à mélanger le liquide contenant le microbe avec l'agent antimicrobien en un rapport prédéfini ou à mélanger le liquide contenant le microbe à une éventuelle concentration avec l'agent antimicrobien à une concentration prédéfinie.

3. Procédé de contrôle selon la revendication 1 ou 2, comprenant en outre :
une sixième étape consistant à introduire dans une base de données une pluralité d'images de microbes qui ont déjà été confirmés comme étant des microbes résistants ;
une septième étape consistant à introduire dans la base de données une pluralité d'images de microbes qui ont déjà été confirmés comme ayant une sensibilité à un agent antimicrobien ;
une huitième étape consistant à obtenir une caractéristique de la pluralité d'images contenues dans la base de données par apprentissage automatique, dans lequel
la cinquième étape consiste à évaluer la sensibilité à des agents chimiques du microbe vis-à-vis de l'agent antimicrobien par comparaison des images des microbes observés dans la troisième étape avec les images contenues dans la base de données sur la base de la caractéristique.

4. Procédé de contrôle selon la revendication 3, dans lequel
la sixième étape et la septième étape sont capables d'ajouter des images à un moment approprié et
la huitième étape consiste à obtenir la caractéristique par apprentissage automatique sur la base des images ajoutées en continu.

5. Procédé de contrôle selon la revendication 1 ou 2, dans lequel la quatrième étape comprend :
une neuvième étape consistant à compter le nombre de microbes dans un champ du microscope lorsque le laps de temps prédéfini s'est écoulé après le mélange du liquide contenant le microbe avec l'agent antimicrobien ;
une dixième étape consistant à discriminer le microbe dont l'aspect a changé par rapport à l'état initial ; et
une onzième étape consistant à calculer un taux d'abondance du microbe dont l'aspect a changé parmi les microbes dans le champ, dans lequel
la cinquième étape consiste à évaluer la sensibilité à des agents chimiques du microbe vis-à-vis de l'agent antimicrobien sur la base du taux d'abondance.

6. Procédé de contrôle selon la revendication 1 ou 2, dans lequel l'aspect du microbe est une forme du microbe et/ou une luminosité de la zone où le microbe est présent.

7. Procédé de contrôle selon la revendication 6, dans lequel la forme du microbe est une forme d'un groupe dans le cas où la pluralité des microbes forment un groupe et/ou une forme de chaque microbe qui est indépendant du groupe.

8. Procédé de contrôle selon la revendication 5, dans lequel
la troisième étape consiste à observer le même échantillon dans une pluralité de champs par le microscope et
la onzième étape consiste à calculer le taux d'abondance du microbe dont l'aspect a changé sur la base du nombre total des microbes dans la pluralité de champs.

9. Procédé de contrôle selon la revendication 1 ou 2, dans lequel
la cinquième étape consiste à évaluer la sensibilité du microbe à l'agent antimicrobien sur la base de la variation du taux d'abondance au cours du temps.

10. Procédé de contrôle selon la revendication 1 ou 2, dans lequel
la quatrième étape consiste à analyser l'aspect par l'obtention d'informations d'aspect du microbe entier obtenues par compilation de données d'aspect de microbes observés et
la cinquième étape consiste à comparer des informations d'aspect dans le cas où l'agent antimicrobien est donné à un microbe qu'une personne prédéfinie a avec des informations d'aspect dans le cas où l'agent antimicrobien n'est pas donné à un microbe que la personne prédéfinie a et évaluer la sensibilité à l'agent antimicrobien du microbe que la personne prédéfinie a sur la base du résultat de la comparaison.

11. Procédé de contrôle selon la revendication 1 ou 2, comprenant en outre :
une douzième étape consistant à obtenir un critère concernant le taux d'abondance de changement d'aspect, établi sur la base d'un agent antimicrobien d'un microbe standard prédéfini et d'une concentration prédéfinie ; dans lequel
la cinquième étape consiste à évaluer la sensibilité d'un microbe qu'une personne prédéfinie a sur la base du critère.

## Patentansprüche

1. Überprüfungsverfahren, welches es ermöglicht, die Empfindlichkeit eines Mikroorganismus gegen ein antimikrobielles Mittel zu überprüfen, wobei es Folgendes umfasst:
einen ersten Schritt, der darin besteht, eine erste Mischprobe einer Flüssigkeit herzustellen, welche einen Mikroorganismus mit dem antimikrobiellen Mittel enthält;
einen zweiten Schritt, der darin besteht, die erste Probe bei einer vorbestimmten Temperatur warmzuhalten und eine zweite Probe, bei welcher es sich um eine Flüssigkeit handelt, die ohne antimikrobielles Mittel einen zweiten Mikroorganismus enthält, welcher vom selben Stamm wie der erste Mikroorganismus ist, bei einer vorbestimmten Temperatur warmzuhalten;
einen dritten Schritt, der darin besteht, einen ersten Mikroorganismus in der ersten Probe zu einer Mehrzahl an vorbestimmten Zeitpunkten mit einem Mikroskop zu beobachten, wobei diese einen Ausgangszustand umfassen, in welchem der erste Mikroorganismus nicht von dem antimikrobiellen Mittel beeinflusst wird, und einen zweiten Mikroorganismus zu mindestens einem der Mehrzahl an vorbestimmten Zeitpunkten mit dem Mikroskop zu beobachten;
einen vierten Schritt, der darin besteht, das Erscheinungsbild des beobachteten ersten Mikroorganismus zu untersuchen und eine erste Abbildung, welche ein Erscheinungsbild des ersten Mikroorganismus betrifft, mit einer zweiten Abbildung zu vergleichen, welche ein Erscheinungsbild des zweiten Mikroorganismus zu mindestens einem der Mehrzahl an vorbestimmten Zeitpunkten betrifft; und
einen fünften Schritt, der darin besteht, die Empfindlichkeit des ersten Mikroorganismus gegen chemische Mittel hinsichtlich des antimikrobiellen Mittels anhand einer Veränderung des Erscheinungsbildes des beobachteten ersten Mikroorganismus zu bewerten,
wobei es sich bei dem Mikroskop um ein Rasterelektronenmikroskop oder ein Rastersondenmikroskop handelt.

2. Überprüfungsverfahren nach Anspruch 1, wobei der erste Schritt darin besteht, die Flüssigkeit, welche den Mikroorganismus enthält, in einem vorbestimmten Verhältnis mit dem antimikrobiellen Mittel zu mischen oder die Flüssigkeit, welche den Mikroorganismus gegebenenfalls in einer Konzentration enthält, mit dem antimikrobiellen Mittel in einer vorbestimmten Konzentration zu mischen.

3. Überprüfungsverfahren nach Anspruch 1 oder 2, wobei es darüber hinaus Folgendes umfasst:
einen sechsten Schritt, der darin besteht, eine Mehrzahl an Abbildungen von Mikroorganismen, welche bereits als resistente Mikroorganismen bestätigt wurden, in eine Datenbank einzufügen;
einen siebten Schritt, der darin besteht, eine Mehrzahl an Abbildungen von Mikroorganismen, welche bereits als empfindlich gegen ein antimikrobielles Mittel bestätigt wurden, in eine Datenbank einzufügen;
einen achten Schritt, der darin besteht, durch selbsttätiges Lernen ein Eigenschaftsmerkmal der Mehrzahl an Abbildungen zu erhalten, welche in der Datenbank enthalten sind, wobei
der fünfte Schritt darin besteht, die Empfindlichkeit des Mikroorganismus gegen chemische Mittel bezüglich des antimikrobiellen Mittels zu bewerten, indem Abbildungen der Mikroorganismen, welche im dritten Schritt beobachtet wurden, anhand des Eigenschaftsmerkmals mit Abbildungen verglichen werden, welche in der Datenbank enthalten sind.

4. Überprüfungsverfahren nach Anspruch 3, wobei
im sechsten Schritt und im siebten Schritt zu einem geeigneten Zeitpunkt Abbildungen hinzugefügt werden können und
der achte Schritt besteht darin, das Eigenschaftsmerkmal durch selbsttätiges Lernen anhand fortlaufend hinzugefügter Abbildungen zu erhalten.

5. Überprüfungsverfahren nach Anspruch 1 oder 2, wobei der vierte Schritt Folgendes umfasst:
einen neunten Schritt, der darin besteht, die Anzahl an Mikroorganismen in einem Sichtfeld des Mikroskops zu zählen, wenn nach dem Mischen der Flüssigkeit, welche den Mikroorganismus enthält, mit dem antimikrobiellen Mittel ein vorbestimmter Zeitraum verstrichen ist;
einen zehnten Schritt, der darin besteht, den Mikroorganismus, dessen Erscheinungsbild sich gegenüber dem Ausgangszustand verändert hat, von anderen zu unterscheiden; und
einen elften Schritt, der darin besteht, unter den Mikroorganismen im Sichtfeld einen Häufigkeitsgrad des Mikroorganismus zu berechnen, dessen Erscheinungsbild sich verändert hat, wobei
der fünfte Schritt darin besteht, die Empfindlichkeit des Mikroorganismus gegen chemische Mittel hinsichtlich des antimikrobiellen Mittels anhand des Häufigkeitsgrads zu bewerten.

6. Überprüfungsverfahren nach Anspruch 1 oder 2, wobei es sich bei dem Erscheinungsbild des Mikroorganismus um eine Gestalt des Mikroorganismus und/oder einen Helligkeitsgrad des Bereichs handelt, in welchem der Mikroorganismus vorhanden ist.

7. Überprüfungsverfahren nach Anspruch 6, wobei es sich bei der Gestalt des Mikroorganismus um eine Gestalt einer Gruppe handelt, wenn die Mehrzahl an Mikroorganismen eine Gruppe bildet, und/oder um eine Gestalt jedes Mikroorganismus, der unabhängig von der Gruppe ist.

8. Überprüfungsverfahren nach Anspruch 5, wobei
der dritte Schritt darin besteht, dieselbe Probe in einer Mehrzahl von Sichtfeldern mit dem Mikroskop zu beobachten, und
der elften Schritt darin besteht, den Häufigkeitsgrad des Mikroorganismus, dessen Erscheinungsbild sich verändert hat, anhand der Gesamtzahl an Mikroorganismen in der Mehrzahl an Sichtfeldern zu berechnen.

9. Überprüfungsverfahren nach Anspruch 1 oder 2, wobei der fünfte Schritt darin besteht, die Empfindlichkeit des Mikroorganismus gegen das antimikrobielle Mittel anhand der Änderung des Häufigkeitsgrads im Zeitverlauf zu bewerten.

10. Überprüfungsverfahren nach Anspruch 1 oder 2, wobei der vierte Schritt darin besteht, das Erscheinungsbild zu untersuchen, indem Informationen zum Erscheinungsbild des Mikroorganismus insgesamt gewonnen werden, wobei diese erhalten wurden, indem Daten zum Erscheinungsbild von beobachteten Mikroorganismen zusammengestellt wurden, und
der fünfte Schritt darin besteht, Informationen zum Erscheinungsbild, wenn das antimikrobielle Mittel zu einem Mikroorganismus gegeben wird, den eine vorbestimmte Person hat, mit Informationen zum Erscheinungsbild zu vergleichen, wenn das antimikrobielle Mittel nicht zu einem Mikroorganismus gegeben wird, den die vorbestimmte Person hat, und die Empfindlichkeit des Mikroorganismus, den die vorbestimmte Person hat, gegen das antimikrobielle Mittel anhand des Vergleichs zu bewerten.

11. Überprüfungsverfahren nach Anspruch 1 oder 2, wobei es darüber hinaus Folgendes umfasst:
einen zwölften Schritt, der darin besteht, ein Kriterium zu erhalten, welches den Häufigkeitsgrad der Änderung des Erscheinungsbildes betrifft, wobei es anhand eines antimikrobiellen Mittels eines vorbestimmten Standard-Mikroorganismus und einer vorbestimmten Konzentration erstellt wurde; wobei
der fünfte Schritt darin besteht, die Empfindlichkeit eines Mikroorganismus, den eine vorbestimmte Person hat, anhand des Kriteriums zu bewerten.

## Claims

1. Checking method for checking the sensitivity of a microbe to an antimicrobial agent, comprising:
a first step of preparing a first sample mixed with a liquid containing a microbe with the antimicrobial agent;
a second step of keeping the first sample warm at a predefined temperature and of keeping a second sample warm at a predefined temperature, this second sample being a liquid containing a second microbe from the same strain as the first microbe but without antimicrobial agent;
a third step of observing a first microbe in the first sample using a microscope at a plurality of predefined times which comprise an initial state in which the first microbe is not affected by the antimicrobial agent and of observing a second microbe using the microscope at at least one of the plurality of predefined times;
a fourth step of analyzing the appearance of the first observed microbe and of comparing a first image regarding an appearance of the first microbe with a second image regarding an appearance of the second microbe at at least one of the plurality of predefined times; and
a fifth step of evaluating the sensitivity of the first microbe to chemical agents with respect to the antimicrobial agent on the basis of a change in appearance of the first observed microbe,
in which the microscope is a scanning electron microscope or a scanning probe microscope.

2. Checking method according to claim 1, in which
the first step consists in mixing the liquid containing the microbe with the antimicrobial agent at a predefined ratio or in mixing the liquid containing the microbe at a possible concentration with the antimicrobial agent at a predefined concentration.

3. Checking method according to Claim 1 or 2, further comprising:
a sixth step of entering into a database a plurality of images of microbes which have already been confirmed as being resistant microbes;
a seventh step of entering into the database a plurality of images of microbes which have already been confirmed as having sensitivity to an antimicrobial agent;
an eighth step of obtaining a characteristic from the plurality of images contained in the database through machine learning, in which
the fifth step consists in evaluating the sensitivity of the microbe to chemical agents with respect to the antimicrobial agent by comparing the images of the microbes observed in the third step with the images contained in the database on the basis of the characteristic.

4. Checking method according to claim 3, in which
the sixth stage and the seventh stage are capable of adding images at an appropriate time; and
the eighth step consists in obtaining the characteristic through machine learning on the basis of the continuously added images.

5. Checking method according to Claim 1 or 2, in which the fourth step comprises:
a ninth step of counting the number of microbes in a field of the microscope once the predefined period of time has elapsed after mixing the liquid containing the microbe with the antimicrobial agent;
a tenth step of discerning the microbe whose appearance has changed with respect to the initial state; and
an eleventh step of calculating an abundance of the microbe whose appearance has changed among the microbes in the field, in which
the fifth step consists in evaluating the sensitivity of the microbe to chemical agents with respect to the antimicrobial agent on the basis of the abundance.

6. Checking method according to Claim 1 or 2, in which the appearance of the microbe is a shape of the microbe and/or a brightness of the region in which the microbe is present.

7. Checking method according to Claim 6, in which the shape of the microbe is a shape of a group in the case of the plurality of microbes form a group and/or a shape of each microbe which is independent of the group.

8. Checking method according to Claim 5, in which
the third step consists in observing the same sample in a plurality of fields using the microscope; and
the eleventh step consists in calculating the abundance of the microbe whose appearance has changed on the basis of the total number of microbes in the plurality of fields.

9. Checking method according to Claim 1 or 2, in which
the fifth step consists in evaluating the sensitivity of the microbe to the antimicrobial agent on the basis of the variation in abundance over time.

10. Checking method according to Claim 1 or 2, in which
the fourth step consists in analysing the appearance by obtaining information on the appearance of the entire microbe obtained by compiling data on the appearance of observed microbes; and
the fifth step consists in comparing appearance information in the case that the antimicrobial agent is given to a microbe that a predefined person has with appearance information in the case that the antimicrobial agent is not given to a microbe that the predefined person has and evaluating the sensitivity of the microbe that the predefined person has to the antimicrobial agent on basis of the result of the comparison.

11. Checking method according to Claim 1 or 2, further comprising:
a twelfth step of obtaining a criterion regarding the abundance of change in appearance, established on the basis of an antimicrobial agent, of a predefined standard microbe and of a predefined concentration; in which
the fifth step consists in evaluating the sensitivity of a microbe that a predefined person has on the basis of the criterion.
